# DEMANDE DE BREVET EUROPEEN

(11) **EP 2 660 311 A1**
(43) Date de publication de la demande: **06.11.2013**
(21) Numéro de dépôt: 13166459.1
(22) Date de dépôt: 03.05.2013
(51) Int. Cl.: C12M 1/107, C12M 1/00

(54) **Reacteur à methanisation à ossature en bois**

(30) Priorité: 04.05.2012 FR 1254098
(71) Demandeur: Enerbiom, 59721 Denain Cedex (FR)
(72) Inventeur: Peultier, Philippe, 59721 Denain (FR)
(74) Mandataire: Bureau Duthoit Legros Associés

(57) **Abrégé**

L'invention concerne un réacteur (1) pour la méthanisation de la biomasse, comprenant :
- un réservoir (2) étanche au gaz comprenant une paroi de fond et des parois latérales, rigides,
- un raccordement (3) de prélèvement de biogaz.

Selon l'invention, tout ou partie des parois rigides (4, 5, 6), en particulier latérales, internes dudit réservoir (2) étanche au gaz est constituée par un complexe constitué essentiellement d'une armature en bois (7), et d'une feuille élastomère (8) recouvrant l'armature en bois (7) de telle façon à protéger le bois de l'armature des gaz de méthanisation, et à assurer l'étanchéité dudit réservoir (2) au gaz et aux liquides.

## Description

L'invention est relative à un réacteur pour la méthanisation de la biomasse.

Dans le domaine de la méthanisation, il est connu des réacteurs de méthanisation, chacun sous la forme d'un garage équipé d'une porte, étanche, permettant le passage d'un véhicule pour le chargement ou le déchargement du réservoir étanche avec de la biomasse. Ce réacteur est équipé d'une conduite de biogaz et d'un dispositif de drainage du jus de suintement. Les performances sont améliorées en maintenant un certain niveau de liquide de suintement dans le réservoir.

Les documents EP-1.301.583 A1 ou FR-2.947.560 décrivent de telles installations.

Les parois de fond et latérales des réservoirs étanches des réacteurs de l'état de la technique sont, à la connaissance de l'inventeur aujourd'hui construites en béton armé, soumises à l'action corrosive des gaz de méthanisation.

La présence en quantité variable de sulfure d'hydrogène dans les gaz de méthanisation corrode fortement le béton des parois. D'après les constatations de l'inventeur, sous l'effet de la corrosion, l'épaisseur des murs en béton peut être réduite de 2 cm par an. Périodiquement, ce type d'installation nécessite une maintenance substantielle, immobilisant l'outil de production. Alternativement, il est connu de protéger les surfaces en béton du réservoir en les recouvrant d'une résine type époxy, augmentant les délais de construction de telles installations, ainsi que leur coût.

L'état de la technique connaît un réacteur de méthanisation construit à partir d'une armature métallique, sous forme de profilés métalliques protégés contre la corrosion par galvanisation ou peinture au zinc. Cette armature métallique est revêtue intérieurement de panneaux en contreplaqué. Cette armature métallique est tapissée intérieurement d'une feuille plastique en matière souple, étanche au gaz.

Un tel réacteur de méthanisation construit à partir d'une armature métallique est de coût de revient élevée, et ne semblerait pas exploité aujourd'hui, à la connaissance de l'inventeur.

Le but de la présente invention est de pallier les inconvénients précités en proposant un réacteur de méthanisation dont le coût de revient est maitrisé et qui peut être érigé rapidement sur site.

D'autre buts et avantages de l'invention apparaîtront au cours de la description qui va suivre qui n'est donnée qu'à titre indicatif et qui n'a pas pour but de la limiter.

Aussi, l'invention est relative à un réacteur pour la méthanisation de la biomasse, comprenant :
- un réservoir, étanche au gaz, comprenant une paroi de fond et des parois latérales, rigides, tout ou partie des parois rigides, en particulier latérales, internes dudit réservoir étanche au gaz étant constituée par un complexe constitué essentiellement d'une armature en bois, et d'une feuille plastique recouvrant l'armature en bois de telle façon à protéger le bois de l'armature des gaz de méthanisation, et à assurer l'étanchéité dudit réservoir au gaz et aux liquides,
- un raccordement de prélèvement de biogaz,
- une installation de drainage du jus de suintement et de recirculation du jus de suintement drainé et dans lequel la base du réservoir étanche au gaz est constituée par une cuvette en béton permettant de créer une garde de liquide de suintement, ladite cuvette formant ladite paroi de fond du réservoir, ainsi que lesdites parois latérales, partiellement sur la hauteur du réservoir étanche, ledit complexe prolongeant les parois latérales en béton de la cuvette.

Selon des caractéristiques optionnelles de l'invention, prises seules ou en combinaison :
- le réacteur présente une installation de chauffage de la biomasse ;
- l'armature en bois comprend des poutres supports, en bois, espacées entre elles, de préférence parallèles entre elles, et des planches, en bois, solidarisées aux poutres supports, disposées jointives entre elles par leurs chants longitudinaux ;

- les poutres supports sont des poteaux, au moins en partie ;
- les parties inférieures de l'armature en bois et de ladite feuille plastique dudit complexe sont noyées dans le béton de la cuvette ;
- ledit réservoir est construit sous la forme d'un garage pour véhicule, étanche aux liquides, l'accès au réservoir étant permis par une porte étanche conçu pour l'admission de la biomasse ;
- la porte est constituée essentiellement par ledit complexe ;
- ladite feuille plastique du complexe recouvre les chants de l'armature en bois de la porte, apte à être déformée élastiquement par gonflage pour assurer l'étanchéité au fluide entre la porte et le cadre de la porte, en position de fermeture de la porte, un circuit d'admission d'air, interne, étant agencé le long des chants périphériques de l'armature en bois de la porte, couplé à un compresseur d'air pour permettre ledit gonflage ;
- la paroi supérieure dudit réservoir étanche au gaz est constituée par une bâche souple, fixée de manière étanche par l'intermédiaire de chacun des ses bords au moyen d'un boudin gonflable de la bâche, ledit boudin gonflable étant destiné à être introduit longitudinalement dans une rainure de forme en queue d'aronde, creusée sur le chant supérieur d'une poutre supérieure de l'armature en bois du complexe, ladite rainure étant éventuellement protégée intimement par la feuille plastique dudit complexe ;
- ladite feuille plastique est une feuille en EPDM ou alternativement ;
- la matière de la feuille plastique est choisie parmi polyéthylène tel que PEHD ou VLDPE, ou encore polypropylène.

L'invention sera mieux comprise à la lecture de la description suivante accompagnée des dessins en annexe parmi lesquelles :
- la figure 1 est une vue schématique, selon une coupe longitudinale, d'un réacteur de méthanisation conforme à l'invention, selon un premier mode de réalisation,
- la figure 1a est une vue de détail de la figure 1,
- la figure 1b est une vue de détail de la figure 1,
- la figure 2 est une vue de dessus d'un agencement de réacteurs, conformes à la revendication 1, disposés côte à côte,
- la figure 2a est une vue de détail illustrant, selon une vue de coupe, une poutre supérieure d'un mur commun à deux réacteurs de l'agencement de la figure 2, selon un premier mode de réalisation,
- la figure 2b est une vue de détail illustrant, selon une vue de coupe, une poutre supérieure d'un mur commun à deux réacteurs de l'agencement de la figure 2, selon un second mode de réalisation,
- la figure 3 est une vue de la porte, et du cadre de porte, du réacteur tel qu'illustré à la figure 1,
- la figure 4a est une vue selon la coupe III-III telle qu'illustrée à la figure 3 selon un premier mode de réalisation de la porte et de son dormant,
- la figure 4b est une vue selon la coupe III-III telle qu'illustrée à la figure 3 selon un deuxième mode de réalisation de la porte et de son dormant,
- la figure 5 est une vue selon une coupe longitudinale d'un réacteur non revendiqué, selon un second mode de réalisation,
- la figure 6 est une vue selon une coupe transversale du réacteur tel qu'illustré à la figure 5.

Aussi l'invention est relative à un réacteur 1 ; 10 pour la méthanisation de la biomasse, comprenant :
- un réservoir 2 étanche au gaz comprenant une paroi de fond et des parois latérales, rigides,
- un raccordement 3 de prélèvement de biogaz,

Selon l'invention, tout ou partie des parois rigides 4, 5, 6 ; 14, 15, 16, en particulier latérales, internes dudit réservoir 2 étanche au gaz est constituée par un complexe constitué essentiellement d'une armature en bois 7, et d'une feuille élastomère 8 recouvrant l'armature en bois de telle façon à protéger le bois de l'armature des gaz de méthanisation, et à assurer l'étanchéité dudit réservoir 2 au gaz et aux liquides.

A cet effet, cette feuille élastomère 8 recouvre donc au moins la face interne de l'armature en bois 7, tournée vers l'intérieur du réservoir, et est soumis à l'action directe des gaz de méthanisation.

Le cas échéant, cette feuille élastomère 8 peut également recouvrir la face externe de l'armature en bois 7, tournée vers l'environnement extérieur afin de la protéger des intempéries afin d'obtenir une étanchéité et une protection totale de l'armature en bois.

De préférence, la feuille élastomère 8 est un thermoplastique, tel que l'EPDM, permettant notamment de souder de manière étanche différentes feuilles 8 du réservoir 2, réalisant ainsi l'étanchéité du réservoir au fluide.

L'armature en bois 7 peut comprendre des poutres supports 70, 71, en bois, espacées entre elles, de préférence parallèles entre elles, et des planches 72, en bois, solidarisées aux poutres supports 70, 71, notamment disposées jointives entre elles par leurs chants longitudinaux, l'agencement de planches 72 réalisant la ou les parois internes, rigides, du réservoir étanche 2. Eventuellement, les planches 72 peuvent être remplacées par des plaques de bois, ou équivalents telles que plaques d'aggloméré.

De préférence, les différentes pièces en bois de l'armature, notamment les planches 72 et les poutres supports 70, 71 peuvent être assemblées par emboitement des pièces entre elles, lesdites pièces présentant à cet effet des parties d'assemblage préalablement usinées en atelier. Ces parties d'assemblage ne sont pas illustrées, car elles sont de pratique courante dans le domaine de la construction bois.

Les différentes pièces de l'armature 7 en bois peuvent ainsi être assemblées sur site en évitant les pièces de fixation rapportées, telles que par exemple boulons, clous, ou similaires.

Selon un mode de réalisation, le réacteur 1 ; 10 peut présenter une installation de chauffage de la biomasse disposée à l'intérieur du réservoir. Cette installation peut, par exemple, comprendre des moyens de chauffage à l'intérieur du réservoir, tels que radiateurs, à l'intérieur du réservoir 2, permettant le chauffage direct de la biomasse à l'intérieur du réservoir étanche 2. Alternativement, l'installation de chauffage permet le chauffage indirect de la biomasse, par exemple par injection d'un liquide chaud dans le réservoir 2, préalablement chauffé à l'extérieur du réservoir étanche 2.

Selon un mode de réalisation, illustré non limitativement aux figures, les poutres supports, au moins en partie, sont verticales formant des poteaux 70.

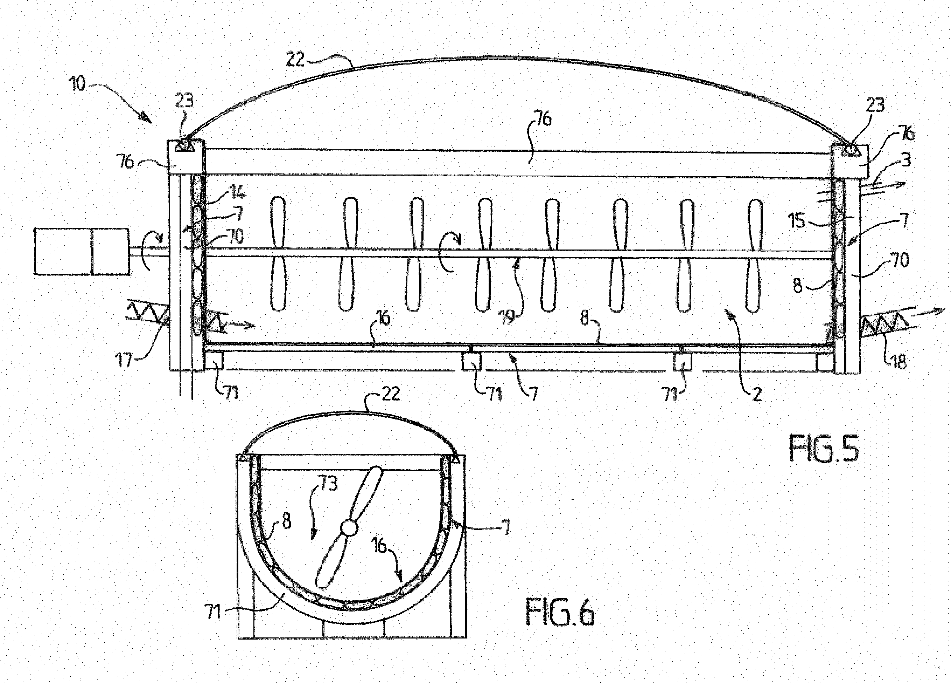

Selon un autre mode de réalisation non revendiqué, illustré non limitativement aux figures 5 et 6, les poutres supports, au moins en partie, sont cintrées 71, l'armature en bois 7 formant un berceau 73 apte à contenir la biomasse. Le berceau 73 peut être fermé à ses extrémités par les parois latérales 14, 15, rigides, verticales, constituées notamment chacune par le complexe.

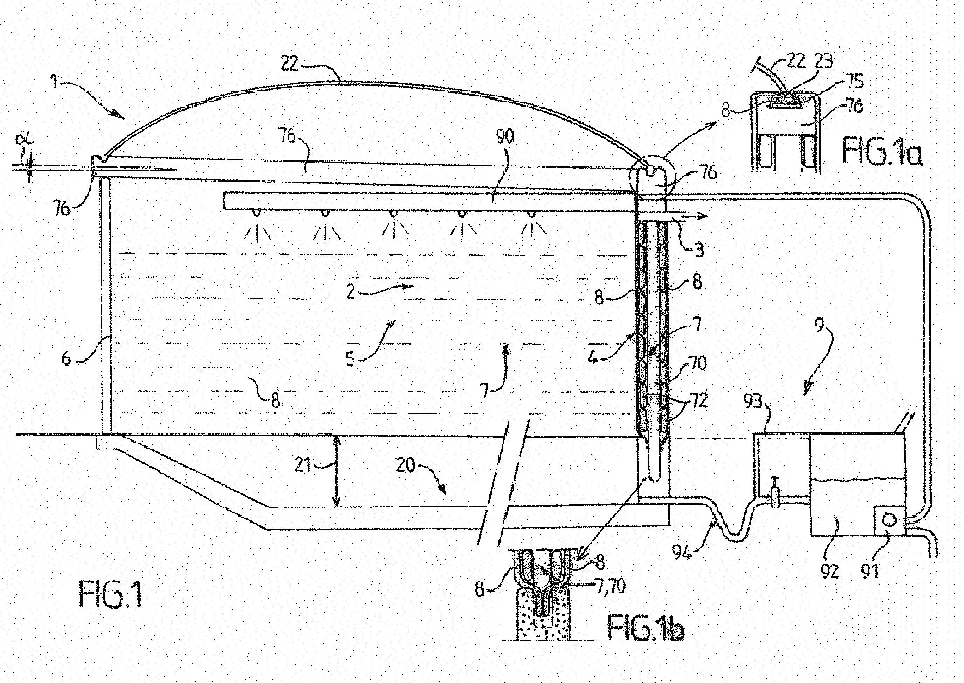

Selon l'invention, illustrée non limitativement des figures 1 à 4, le réacteur 1 peut présentant une installation 9 de drainage du jus de suintement et de recirculation du jus de suintement drainé.

L'installation 9 peut ainsi présenter une conduite apte à récupérer le jus en partie inférieure du réservoir étanche 2 et à recirculer ce jus en partie haute du réservoir jusqu'à une rampe de pulvérisation 90, grâce à une pompe 91. Ce circuit peut comprendre un moyen de chauffage du jus recirculé, constituant ladite installation de chauffage de la biomasse à l'intérieur du réservoir.

Le circuit de recirculation peut comprendre un réservoir de pompage 92, étanche à l'air afin que se maintienne une réaction anaérobie. A cet effet, un siphon 94 peut être prévu entre le réservoir étanche 2 et ledit réservoir de pompage 92. Le moyen de chauffage peut être un radiateur tel qu'une résistance électrique, interne au réservoir 92.

Selon l'invention, illustrée non limitativement aux figures 1 à 4, la base du réservoir étanche 2 au gaz est constituée par une cuvette 20 en béton armé permettant de créer une garde de liquide 21 de suintement, notamment défini par une surverse 93 de l'installation 9. Cette cuvette 20 peut notamment présenter un plan incliné formant une rampe pour un véhicule, et une paroi horizontale tels que définis dans la demande FR 09/03249, du présent demandeur, qui est introduite par référence.

Cette cuvette 20 forme ladite paroi de fond du réservoir, ainsi que lesdites parois latérales 4, 5, partiellement sur la hauteur de la cuvette, ledit complexe prolongeant verticalement les parois latérales en béton de la cuvette, ledit complexe étant fixé à la maçonnerie.

Avantageusement, les parties inférieures de l'armature en bois 7, en particulier des poteaux 70, et la partie inférieure de ladite feuille élastomère 8 peuvent être noyées dans le béton de la cuvette 20 afin d'assurer une fixation robuste du complexe au béton, ainsi que l'étanchéité au fluide entre la maçonnerie et le complexe (voir figure 1b).

Selon une autre alternative l'armature bois du complexe peut être fixée aux parois latérales de la cuvette par l'intermédiaire de supports tels que cavalier.

Ledit réservoir 1 peut être construit sous la forme d'un garage pour véhicule, étanche aux liquides, l'accès au réservoir étant permis par une porte étanche 6 conçue pour l'admission de la biomasse. Avantageusement, cette porte étanche 6 formant l'une des parois latérales du réservoir (en position de fermeture) peut être également constituée par le complexe.

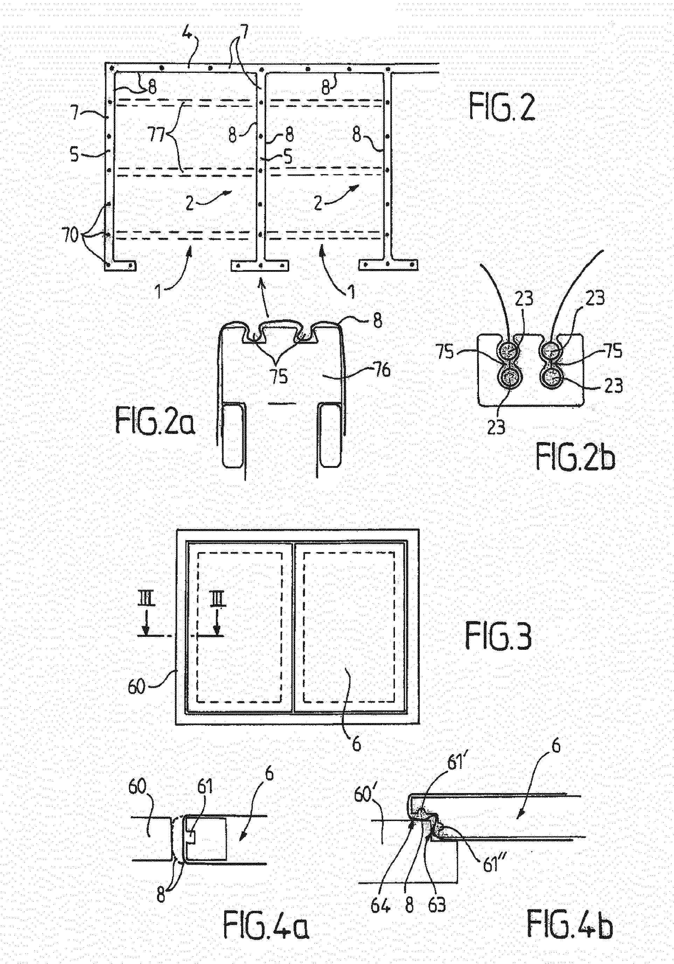

Selon un mode de réalisation illustré non limitativement aux figures 3 et 4, ladite feuille élastomère 8 du complexe peut recouvrir les chants de l'armature en bois de la porte 6 étanche.

Avantageusement, cette feuille élastomère 8 peut être déformée élastiquement par gonflage afin d'assurer l'étanchéité au fluide entre la porte 6 (ouvrant) et le cadre 60 de la porte (dormant).

A cet effet, un circuit 61 d'admission d'air, interne audit complexe, est agencé le long des chants périphériques de l'armature en bois de la porte 6, le circuit étant constitué notamment par une rainure de l'armature en bois 7, ladite rainure débouchant directement sous la feuille élastomère 8.

Ce circuit 61 est couplé à un compresseur d'air pour permettre ledit gonflage de la feuille 8 élastomère au niveau du chant de la porte. Lorsque gonflée, telle qu'illustrée en pointillés à la figure 4a, la feuille gonflée vient appuyer sur le chant du cadre 60 de porte afin de réaliser l'étanchéité entre la porte 6 et son cadre 60. Le dégonflage de la feuille permet au contraire de créer un jeu entre la porte 6 et son cadre facilitant l'ouverture de la porte 6.

Selon un mode de réalisation illustré à la figure 4b, la porte 6' peut présenter un épaulement périphérique 62 destiné à appuyer simultanément sur la tranche et la paroi intérieure du cadre de porte 60'. La porte 6' s'ouvrira à l'intérieur du réservoir 1. Lors de la méthanisation le réservoir 1 étant à une pression supérieure à la pression atmosphérique, ledit épaulement périphérique 62 sera alors plaqué sur le cadre de porte 60', sous l'effet de la différence de pression régnant entre le réservoir et l'extérieur.

Un double circuit 61',61" d'admission d'air, interne audit complexe de la porte 60' peut être agencé le long dudit épaulement périphérique et permettre le gonflage de la feuille, d'une part, entre la tranche 63 du cadre de porte 60' et ledit épaulement périphérique 62, et d'autre part, entre la paroi intérieure 64 du cadre de porte 60' et ledit épaulement périphérique 62, assurant ainsi une double étanchéité à la fermeture. Selon un mode de réalisation, la paroi supérieure dudit réservoir 2 étanche au gaz peut être constituée par une bâche 22, fixée de manière étanche par l'intermédiaire de chacun des ses bords au moyen d'un boudin gonflable 23 de la bâche 22.

Le boudin, lorsque dégonflé, peut être introduit longitudinalement dans une rainure de blocage 75, notamment de forme en queue d'aronde, creusée sur le chant supérieur d'une poutre supérieure 76, en bois, de l'armature bois 7. Lorsque gonflé, ce boudin gonflable 23 se bloque dans la rainure 75 et assure l'étanchéité entre le complexe et la bâche 23.

Afin d'augmenter la résistance à l'arrachement, la rainure de blocage 75 peut présenter deux zones de blocage, l'une au dessus de l'autre, pour le blocage respectif de deux boudins 23 périphériques juxtaposés (voir figure 2b).

De préférence, la rainure de blocage 75 est protégée intimement par la feuille élastomère 8 dudit complexe. De préférence, les poutres supérieures 76 de l'armature en bois, notamment longitudinales au réacteur 1 ; 10, sont inclinées par rapport à l'horizontal de telle façon à favoriser l'écoulement de l'eau. L'inclinaison α est, par exemple, comprise entre 1 % et 5%, tel que 2%, évitant ainsi la stagnation de l'eau de pluie sur le dessus des poutres supérieures 76.

Selon un mode de réalisation, non limitatif, illustré aux figures 1 à 4, le réservoir étanche 2 peut présenter, vue de dessus, une section rectangulaire, comportant quatre parois latérales 4, 5, 6, deux à deux parallèles, l'une d'entre elles étant constituée par la porte 6 (ou 6'), l'autre par une paroi 4 opposée à cette porte 6 (ou 6'), ainsi que deux parois longitudinales 5 deux à deux parallèles. Ces parois longitudinales 5, constituées en partie supérieure par le complexe peuvent être reliées entre elles, à leur partie supérieure, au moyen de traverses 77 en bois, elles mêmes recouvertes de manière étanche d'une feuille élastomère 8.

Selon un autre mode de réalisation non revendiqué, illustré aux figures 5 et 6, l'armature en bois 7 forme au moins un berceau 73 apte à contenir de la biomasse. Les planches 72 sont disposées longitudinalement au berceau 73, fixées sur la partie concave des poutres cintrées 71 afin de réaliser la paroi interne du berceau 73, recouverte de la feuille élastomère 8.

Les extrémités du berceau 73 peuvent être fermées, chacune de manière étanche par une paroi latérale 14 ou 15. Chaque paroi 14 ou 15 peut être constituée du complexe.

Selon ce mode de réalisation, ce réservoir étanche 2 peut présenter une ouverture d'alimentation 17 de biomasse, à l'une des extrémités du berceau 73, notamment alimentée en biomasse par une vis de transport, et une sortie 18 de biomasse à l'autre des extrémités dudit berceau 73, notamment pourvu d'une vis de transport pour l'évacuation de la biomasse.

Un dispositif 19 de convoyage mécanique est disposé dans le berceau 73, suivant la longueur du berceau, apte à assurer le transfert de la biomasse depuis l'ouverture d'alimentation 17 jusqu'à ladite sortie 18 de biomasse. Tel qu'illustré aux figures 5 et 6, il peut notamment s'agir d'un rotor pourvu de pales réparties sur la longueur et la circonférence du rotor, assurant le mélange et l'avancement de la biomasse lors de sa rotation.

La feuille élastomère peut être de l'EPDM tel que décrit plus haut. Plus généralement la feuille élastomère peut être remplacée par une feuille plastique. La matière de la feuille plastique peut être choisie parmi le polyéthylène tel que PEHD ou VLDPE, ou encore le polypropylène.

Naturellement d'autres modes de réalisation auraient pu être envisagés par l'homme du métier sans pour autant sortir du cadre de l'invention définie par les revendications ci-après.

### NOMENCLATURE

1 Réacteur de méthanisation (Premier mode de réalisation),
2 Réservoir étanche,
4, 5 Parois latérales (Réacteur 1),
6 Porte étanche (formant paroi latérale du réacteur 1),
7 Armature bois,
8 Feuille élastomère (exemple EPDM),
9 Installation de drainage et de recirculation du jus de suintement,
10 Réacteur de méthanisation (Second mode de réalisation),
14, 15, 16 Parois latérales (Réacteur 10),
17 Ouverture d'alimentation (Réacteur 10),
18 Sortie pour biomasse (Réacteur 10),
20 Cuvette en béton (Réacteur 1),
21 Garde de liquide de jus de suintement,
22 Bâche souple (formant la paroi supérieure du réacteur 1 ou 10),
23 Boudin gonflable (bâche 22),
60, 60' Cadre de porte (Porte 6, 6'),
61, 61', 61" Circuit d'admission d'air (Porte 6 ou 6'),
62 Epaulement périphérique (Porte 6'),
63 Tranche (Cadre de porte 60'),
64 Paroi intérieure (Cadre de porte 60'),
70 Poutres supports verticales (Poteaux de l'armature en bois),
71 Poutres supports cintrées (Armature bois),
72 Planches (Armature en bois),
73 Berceau,
75 Rainure de blocage (Poutre supérieure 76),
76 Poutre supérieure,
77 Traverses.

## Revendications

1. Réacteur (1; 10) pour la méthanisation de la biomasse, comprenant :
- un réservoir (2) étanche au gaz comprenant une paroi de fond et des parois latérales, rigides, tout ou partie des parois rigides (4, 5, 6 ; 14, 15, 16), en particulier latérales, internes dudit réservoir (2) étanche au gaz étant constituée par un complexe constitué essentiellement d'une armature en bois (7), et d'une feuille plastique (8) recouvrant l'armature en bois (7) de telle façon à protéger le bois de l'armature des gaz de méthanisation, et à assurer l'étanchéité dudit réservoir (2) au gaz et aux liquides,
- un raccordement (3) de prélèvement de biogaz,
- une installation (9) de drainage du jus de suintement et de recirculation du jus de suintement drainé et dans lequel la base du réservoir étanche au gaz est constituée par une cuvette (20) en béton permettant de créer une garde de liquide (21) de suintement, ladite cuvette (20) formant ladite paroi de fond du réservoir, ainsi que lesdites parois latérales, partiellement sur la hauteur du réservoir étanche (2), ledit complexe prolongeant les parois latérales en béton de la cuvette.

2. Réacteur (1 ; 10) selon la revendication 1 présentant une installation de chauffage de la biomasse à l'intérieur du réservoir.

3. Réacteur (1 ; 10) selon la revendication 1 ou 2, dans lequel l'armature en bois (7) comprend des poutres supports (70, 71), en bois, espacées entre elles, de préférence parallèles entre elles, et des planches (72), en bois, solidarisées aux poutres supports, disposées jointives entre elles par leurs chants longitudinaux.

4. Réacteur (1 ; 10) selon la revendication 3, dans lequel les poutres supports sont des poteaux verticaux (70) au moins en partie.

5. Réacteur (1)) selon l'une des revendications 1 à 4, dans lequel les parties inférieures de l'armature en bois (7) et de ladite feuille plastique (8) dudit complexe sont noyées dans le béton de la cuvette (20).

6. Réacteur (1) selon l'une des revendications 1 à 5, dans lequel ledit réservoir est construit sous la forme d'un garage pour véhicule, étanche aux liquides, l'accès au réservoir étant permis par une porte étanche (6) conçu pour l'admission de la biomasse.

7. Réacteur selon la revendication 6, dans lequel la porte (6) est constituée essentiellement par ledit complexe.

8. Réacteur selon la revendication 7, dans lequel ladite feuille plastique (8) du complexe recouvre les chants de l'armature en bois de la porte (6), apte à être déformée élastiquement par gonflage pour assurer l'étanchéité au fluide entre la porte (6 ; 6') et le cadre (60, 60') du dormant de la porte, un circuit (61; 61',) d'admission d'air, interne, étant agencé le long des chants périphériques de l'armature en bois de la porte (6), couplé à un compresseur d'air pour permettre ledit gonflage.

9. Réacteur selon la revendication 7 ou 8, dans lequel la porte (6') présente un épaulement périphérique (62) destiné à appuyer simultanément sur la tranche (63) et la paroi intérieure (64) du cadre (60') du dormant de porte, la porte (6') s'ouvrant à l'intérieur du réservoir (1).

10. Réacteur (1 ; 10) selon l'une des revendications 1 à 9, dans lequel la paroi supérieure dudit réservoir (2) étanche au gaz est constituée par une bâche souple (22), fixée de manière étanche par l'intermédiaire de chacun des ses bords au moyen d'au moins un boudin gonflable (23) de la bâche, ledit boudin gonflable étant destiné à être introduit longitudinalement dans une rainure (75) de blocage, creusée sur le chant supérieur d'une poutre supérieure (76) de l'armature en bois (7) du complexe, ladite rainure étant éventuellement protégée intimement par la feuille plastique (8) dudit complexe.

11. Réacteur selon l'une des revendications 1 à 10, dans lequel ladite feuille plastique (8) est une feuille en EPDM.

12. Réacteur selon l'une des revendications 1 à 10, dans lequel la matière de la feuille plastique (8) est choisie parmi :
- polyéthylène tel que PEHD ou VLDPE
- polypropylène.
